# EUROPEAN PATENT SPECIFICATION

(11) **EP 2 286 739 B1**
(45) Date of publication and mention of the grant of the patent: **02.01.2013**
(21) Application number: 10014491.4
(22) Date of filing: 08.07.2009
(51) Int. Cl.: A61B 17/11, A61F 2/82, A61B 19/00

(54) **Anastomosis sheath**
Anastomosedecke
Gaine d'anastomose

(30) Priority: 09.07.2008 US 79200 P; 17.06.2009 US 486346
(43) Date of publication of application: 23.02.2011
(62) Divisional of application: 09251764.8
(73) Proprietor: Covidien LP, Mansfield, MA 02048 (US)
(72) Inventor: Stopek, Joshua, Guilford CT 06437 (US); Jones, Jacqueline, Hamden CT 06518 (US); Elachchabi, Amin, Hamden CT 06518 (US)
(74) Representative: Soames, Candida Jane

(56) References cited:
- EP-A- 0 824 901
- US-A1- 2005 182 484

## Description

### Technical Field

The present disclosure relates to a sheath for use with an anastomosis to prevent fluid leaks, and more particularly to a sheath made with at least one biodegradable and non-biodegradable portion.

### Background

An anastomosis, or the joining of two vessels, such as portions of the esophagus, colon, or other parts of the digestive tract, is a common procedure. Sometimes, however, there are complications associated with the anastomotic site.

Specific patient populations such as patients with diabetes T1, T2, or other immuno-compromised patients (such as chemotherapy patients) are more prone to anastomotic leaks. These patient populations have longer healing profiles and sometimes weaker immune systems and these factors may lead to an increase in leak occurrence. Unfortunately, in most cases, anastomotic leaks are not detected until clinical symptoms present themselves.

Currently, sheaths are attached to an anastomotic site, and the sheaths either have to be surgically removed or sloughed off from a body lumen wall. When staples or sutures separate from the body lumen wall, this separation may cause damage to the body lumen wall.

While current anastomotic devices and surgical methods perform satisfactorily, it would be advantageous to provide a device to reduce the risks associated with anastomotic leaks.
US 2005/182484 describes a hybrid graft for vascular use, which comprises a synthetic material portion and an extracellular matrix portion (ECM). Such a graft may be used in the treatment of a vascular anastomosis. No component of the vascular graft according to US 2005/182484 detaches and passes into the vascular system when in use.

### SUMMARY

A sheath is described herein which provides for protection of an anastomosis and a reduction in anastomotic leaks. The sheath includes a sleeve defining a passage and the sleeve includes at least one biodegradable portion and at least one non-biodegradable portion. The at least one biodegradable portion is affixed to a body lumen distal to an anastomotic site with the at least one non-biodegradable portion extending proximally from the biodegradable portion. In some embodiments, the anastomotic site is intestinal or vascular.

The biodegradable portion is selected from the group consisting of synthetic and natural materials. Suitable synthetic materials can include polyurethanes, polyhydroxybuterates, polylactides, polyglycolides, polydioxanones, polyanhydrides, poly (amino acids), poly (ortho esters), polycaprolactones and combinations thereof. Suitable natural materials can include collagen, cellulose, polysaccharides, hyaluronic acid, and combinations thereof. The sheath is capable of passage from the body lumen upon degradation of the at least one biodegradable portion.

The non-biodegradable portion is preferably selected from the group consisting of polyolefins, fluorinated polymers, urethanes, polyesters, nylons, polyaramids, silicones, and combinations thereof.

In certain embodiments, sheaths according to the present disclosure may be constructed at least in part using shape memory polymers. Suitable polymers used to prepare hard and soft segments of shape memory polymers include polycaprolactone, polydioxanone, lactide (poly lactic acid), glycolide (poly glycolic acid), polyacrylates, polyamides, polysiloxanes, polyurethanes, polyether amides, polyurethane/ureas, polyether esters, and urethane/butadiene copolymers and combinations thereof.

Methods for joining the non-biodegradable portion and the biodegradable portion of the sheath are selected from the group consisting of melt pressing, heat melding, gluing, solvent welding, over-molding, suturing, stapling, tacking, and combinations thereof.

The sheath may be a film and also may be tubular in shape. The sheath may further comprise an active agent, a lubricious coating, or an image-enhancing agent.

Once the sheath is inserted into the body, it may be affixed to the anastomotic site using a method selected from the group consisting of sutures, staples, tacks, glues, stents, rings, and combinations thereof.

The sheath includes a sleeve defining a passage; the sleeve has at least one biodegradable portion and at least one non-biodegradable portion. The at least one non-biodegradable portion is affixed to a body lumen proximal to an anastomotic site and the at least one biodegradable portion extends distally from the non-biodegradable portion. The sheath is capable of removal from the body lumen. In an alternate embodiment, the sheath further comprises a second non-biodegradable portion, which attaches to a distal end of the biodegradable portion. The second non-biodegradable portion extends distally from the anastomotic site.

Another embodiment is also disclosed in which the majority of the total length of the sheath comprises at least one non-biodegradable portion.

### BRIEF DESCRIPTION OF THE DRAWINGS

The illustrative embodiments described herein will become more readily apparent from the following description, reference being made to the accompanying drawings in which:

FIG. 4 shows a perspective cross-sectional view of a second embodiment of a sheath in accordance with the present disclosure;

FIG. 5 shows a perspective view of a third embodiment of a sheath in accordance with the present disclosure; and,

FIG. 6 shows a perspective view of another embodiment of a sheath in accordance with the present disclosure.

### DETAILED DESCRIPTION OF EMBODIMENTS

The present disclosure is directed to a biocompatible anastomotic sheath. The sheath is a sleeve including at least one biodegradable and one non-biodegradable portion. The sheath is affixed to a body lumen, proximal to an anastomotic site, to enable fluids to bypass the anastomosis while preventing leakage of tissue luminal contents. Upon degradation of the biodegradable portion, the sheath is expelled from the body through a natural passageway.

In the description that follows, the term "body lumen" as used herein, means inner open space or cavity of a tubular organ, such as a blood vessel, intestine, or esophagus. The term "biodegradable" as used herein refers to materials which decompose, or lose structural integrity under body conditions (e.g., enzymatic degradation or hydrolysis). The term "proximal" as used herein, means the portion of the sheath which is nearer to the user, while the term "distal" refers to the portion of the sheath which is further away from the user.

The sheath, at least in part, is comprised of biodegradable materials which may be synthetic or natural materials. Suitable synthetic biodegradable materials include polymers such as those made from lactide, glycolide, caprolactone, valerolactone, carbonates (e.g., trimethylene carbonate, tetramethylene carbonate, and the like), dioxanones (e.g., 1,4-dioxanone), 1,dioxepanones (e.g., 1,4-dioxepan-2-one and 1,5-dioxepan-2-one), ethylene glycol, ethylene oxide, esteramides, γ-hydroxyvalerate, β-hydroxypropionate, alpha-hydroxy acid, hydroxybuterates, poly (ortho esters), hydroxy alkanoates, tyrosine carbonates, polyimide carbonates, polyimino carbonates such as poly (bisphenol A-iminocarbonate) and poly (hydroquinone-iminocarbonate), polyurethanes, polyanhydrides, polymer drugs (e.g., polydiflunisol, polyaspirin, and protein therapeutics) and copolymers and combinations thereof. Suitable natural biodegradable polymers include collagen, cellulose, poly (amino acids), polysaccharides, hyaluronic acid, gut, copolymers and combinations thereof.

Suitable non-biodegradable materials which may be used to construct the sheath include fluorinated polymers (e.g., fluoroethylenes, propylenes, fluoroPEGs), polyolefins such as polyethylene, polyesters such as poly ethylene terepththalate (PET), nylons, polyamides, silicones, ultra high molecular weight polyethylene (UHMWPE), polybutesters, polyaryletherketone, copolymers and combinations thereof. Additionally, non-biodegradable polymers and monomers may be combined with each other and may also be combined with various biodegradable polymers and monomers to create a composite sheath.

Suitable materials of the present disclosure can be processed within the purview of those skilled in the art including, but not limited to extrusion, injection molding, compression molding, blow molding, film blowing, thermoforming, calendaring, spinning and film casting.

In some embodiments, the biodegradable and non-biodegradable portions of the sheath are each films, wherein each film portion can be porous or semi-permeable to enable or restrict oxygen and nutrient transport. In some embodiments, semi-permeable or controlled permeability properties along some or all of the sheath's length allow absorption of certain nutrients at the appropriate location in the body lumen wall. For example, when the sheath is used in the intestines, nutrient absorption at a specific location along the gastrointestinal tract is desirable to avoid malabsorption. Alternately, a specific length or the entire length of the sheath may be non-porous or impermeable. It will be understood that other embodiments are within the purview of those skilled in the art and are within the context of the present disclosure. For example, alternate embodiments such as foams or woven fibers may be preferred to films when the sheath's surface area is used to alter the degradation times and profiles. It should also be understood that the above discussion including a structure's permeability, for example, a semi-permeable membrane or controlled permeability, are not limited to a sleeve and may also include additional parts of the sheath.

Once processed, biodegradable and non-biodegradable portions may be joined together to create a sheath using various mechanical and chemical means within the purview of those skilled in the art. Methods include, but are not limited to, heat melding/melt pressing, glues/adhesives, solvent welding, ultraviolet radiation, ultrasonic energy, extrusion (e.g., coextrusion or compound extrusion), over molding, suturing, stapling and tacking. In some embodiments, glues/adhesives include but are not limited to cyanoacrylates, urethanes, and siloxanes. The biodegradable and non-biodegradable portions once joined, create a sheath wherein the non-biodegradable and biodegradable portions remain joined until the biodegradable portion degrades in situ.

Suitable solvents for use in solvent welding include but are not limited to polar and non-polar solvents such as alcohols, e.g., methanol, ethanol, propanol, chlorinated hydrocarbons (such as methylene chloride, chloroform, 1, 2-dichloro-ethane), and aliphatic hydrocarbons such as hexane, heptene, and ethyl acetate.

Optionally, the sheath can include coatings on its interior and/or exterior to enhance the surface properties in clinically relevant manners. As used herein, the term "coating" is not limited to liquids and may also include waxes and solids. For example, a parylene coating may be used to increase the chemical resistance of the sleeve material. In other embodiments, lubricious coatings may be applied which aid in nutrient passage through the sheath such as polyethylene glycols. Coatings can be applied using any method within the purview of those skilled in the art.

Additionally, any part of the sheath including the biodegradable portions and the non-biodegradable portions may include biologically acceptable additives such as plasticizers, antioxidants, dyes, image-enhancing agents, dilutants, bioactive agents such as pharmaceutical and medicinal agents, and combinations thereof which can be coated on the sheath or impregnated within a resin or polymer.

Medicinal agents which may be incorporated into the sheath include antimicrobial agents, anti-virals, anti-fungals, and the like. Antimicrobial agents as used herein is defined by an agent which by itself or through assisting the body (immune system) helps the body destroy or resist microorganisms which may be pathogenic (disease causing). The term "antimicrobial agent" includes antibiotics, quorum sensing blockers, surfactants, metal ions, antimicrobial proteins and peptides, antimicrobial polysaccharides, antiseptics, disinfectants, anti-virals, anti-fungals, and combinations thereof.

Examples of suitable antiseptics and disinfectants which may be combined with the present disclosure include hexachlorophene, cationic biguanides like chlorohexadine and cyclohexidine, iodine and iodophores like povidone-iodine, halo-substituted phenolic compounds like PCMX (e.g., p-chloro-m-xylenon) and triclosan (e.g., 2,4,4'-trichloro-2'hydroxydiphenylether), furan medical preparations like nitrofurantoin and nitrofurazone, methanamine, aldehydes like gluteraldehyde and formaldehyde, alcohols, combinations thereof, and the like.

Classes of antibiotics that can be combined with the present disclosure include tetracyclines like minocycline, rifamycins like rifampin, macrolides like erythromycin, penicillins like nafcillin, cephalosporins like cefazolon, beta-lactam antibiotics like imipenen and aztreonam, aminoglycosides like gentamicin and TOBRAMYCIN®, chloramphenicol, sulfonamides like sulfamethoxazole, glycopeptides like vancomycin, quilones like ciproflaxin, fusidic acid, trimethoprim, metronidazole, clindamycin, mupirocin, polyenes like amphotericin B, azoles like fluconazole, and beta-lactam inhibitors like sublactam. Other antimicrobials which may be added include, for example, antimicrobial peptides and/or proteins, antimicrobial polysaccharides, quorum sensing blockers, anti-virals, metal ions such as ionic silver and ionic silver glass, surfactants, chemotherapeutic drug, telomerase inhibitors, other cyclic monomers including 5-cyclic monomers, mitoxantrone, and the like.

In some embodiments, suitable bioactive agents which may be used include colorants, dyes, preservatives, protein and peptide preparations, protein therapeutics, polysaccharides such as hyaluronic acid, lectins, lipids, probiotics, angiogenic agents, anti-thrombotics, anti-clotting agents, clotting agents, analgesics, anesthetics, wound repair agents, chemotherapeutics, biologics, anti-inflammatory agents, anti-proliferatives, diagnostic agents, antipyretic, antiphlogistic and analgesic agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, antiviral agents, dysuric agents, brominated or halogenated furanones, and the like. In embodiments, polymer drugs, e.g., polymeric forms of such compounds, for example, polymeric antibiotics, polymeric antiseptics, polymeric chemotherapeutics, polymeric anti-proliferatives, polymeric antiseptics, polymeric non-steroidal anti-inflammatory drugs (NSAIDS), and the like may be utilized and combinations thereof.

In certain embodiments, sheaths of the present disclosure may contain suitable medicinal agents such as viruses and cells, peptides, polypeptides and proteins, analogs, muteins, and active fragments thereof, such as immunoglobulins, antibodies (monoclonal and polyclonal), cytokines (e.g., lymphokines, monokines, chemokines), blood clotting factors, hemopoietic factors, interleukins (IL-2, IL-3, IL-4, IL-6), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens), somatostatin, antigens, blood coagulation factors, growth factors, protein inhibitors, protein antagonists, and protein agonists, nucleic acids, such as antisense molecules, DNA, RNA, oligonucleotides, polynucleotides and ribozymes, and combinations thereof.

In some embodiments, additives such as image-enhancing agents (e.g., contrast agents) and more specifically, radiopaque markers, may be incorporated into the sheath. These image-enhancing agents enable visualization of the sheath (against surrounding tissue), when imaged or scanned through different filters such as MRI, X-ray, fluoroscopy, CT, various light sources, and the like. In order to be opaque, the sheath must be made from a material possessing a radiographic density higher than the surrounding host tissue and have sufficient thickness to affect the transmission of x-rays to produce contrast in the image. Useful image-enhancing agents include but are not limited to radiopaque markers such as tantalum, barium sulfate, bismuth trioxide, bromine, iodide, titanium oxide, zirconium, barium, titanium, bismuth, iodine, nickel, iron, silver, and combinations thereof. In some embodiments, compounds such as tantalum, platinum, barium and bismuth may be incorporated into the sheath. Often image-enhancing agents are not bioabsorbable or biodegradable but are excreted from the body or stored in the body.

In some embodiments, image-enhancing agents may be compounded into the materials (e.g., resin) as filler prior to processing including extrusion or molding. These agents may be added in various concentrations to maximize polymer processing while maximizing the mechanical properties of the sheath. The biocompatible agents can be added in quantities sufficient to enhance radiopacity while maintaining the polymer's properties. In certain embodiments, image-enhancing agents may be incorporated into the biodegradable portion, enabling surgeons to visualize when the biodegradable portion has degraded and the non-biodegradable portion of the sheath has passed.

Methods for combining the above mentioned bioactive agents with compositions of the present disclosure are within the purview of those skilled in the art and include, but are not limited to mixing, blending, compounding, spraying, wicking, solvent evaporating, dipping, brushing, vapor deposition, coextrusion, capillary wicking, film casting, molding and the like. Additionally, solvents may be used to incorporate various agents into the composite device. Suitable solvents include those listed above.

Sheaths of the present disclosure may be affixed to the body lumen wall proximal to an anastomosis site before, during, or after anastomosis creation. In several embodiments, sheaths may be inserted into the body cavity and body lumen either in an expanded form or a collapsed or compressed position. Once attached to the body lumen, the sheath enables fluids to bypass the anastomosis, reducing amount of fluid contact with anastomosis and therefore reducing the potential for anastomotic fluid leaks into the surrounding environment. In some embodiments, an inverted sheath may be inserted into the body lumen, attached to the body lumen wall, and then extended through the body lumen, distal to the anastomotic site. In one example, when used in the colon or intestinal tract, sheaths of the present disclosure may be useful for limiting nutrient absorption for controlling T2 diabetes.

Various degradation profiles and times are contemplated for the biodegradable portion. Degradation times and profiles may be controlled by factors such as material selection and sheath surface area. In a preferred embodiment, the biodegradable portion has a persistence time of about 1 day to about 12 weeks, in a more preferred embodiment about 3 days to about 21 days. In preferred embodiments, mass loss corresponds closely to strength loss of the biodegradable portion, hence when the sheath loses mechanical properties of the biodegradable portion, the mass of remaining biodegradable portion will be minimal such as to mitigate inflammation and encapsulation. Once the biodegradable portion loses significant mass and/or strength, the non-biodegradable portion may separate and pass naturally through the body, and exit the body through a natural orifice.

In preferred embodiments, the sheath is tubular-shaped as shown in FIGS. 1-6, although other shapes are envisioned such as elliptical, conical and rectangular. Shapes of sheaths in addition to concavity of sheaths of the present disclosure may vary depending on factors such as the method of use and patient anatomy.

Sheaths of the present disclosure can be affixed to the lumen wall proximal to an anastomosis site using various methods including but not limited to staples, sutures, glues, clips, or tacks. Once attached, the sheath enables fluids to bypass the anastomosis, reducing amount of fluid contact with anastomosis, therefore, reducing the potential for anastomotic fluid to leak into the surrounding body cavity. It is important to note that the biodegradation of the sheath can be tailored to correspond with a wound healing profile of a specific body lumen, promoting maximal wound healing prior to degradation and release or removal of the non-biodegradable portion.

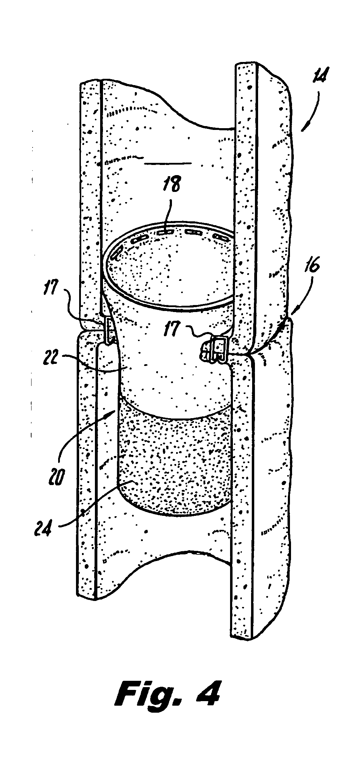

As illustrated in FIG. 4, an embodiment of a sheath 20 includes a non-biodegradable portion 22 and a biodegradable portion 24. A proximal portion of the sheath 20 is attached proximally to anastomosis site 16 using sutures 18, which may be bioabsorbable or non-absorbable. An anastomosis may be created using traditional methods including surgical staples 17, as shown by example in FIG. 4.

The non-biodegradable portion 22 may have preferred mechanical or chemically properties for attachment to the body lumen 14. By way of example, in the intestines, a silicone material may be employed as the non-biodegradable portion 22. Silicone is relatively stiff, tear resistant and easy to suture through, which, in some embodiments, makes silicone a preferable material for the non-biodegradable portion 22. In this embodiment, the non-biodegradable portion 22 is attached at a position spaced apart from an anastomosis site, and a biodegradable portion 24 is attached distal to the non-biodegradable portion 22 as shown in FIG. 4. The degradation of the distal biodegradable portion 24 would minimize material left in the body lumen, inciting less of an inflammatory response. In some embodiments, the non-biodegradable portion 22 may later be removed through surgical means or it may detach from the body lumen wall and naturally pass through the body.

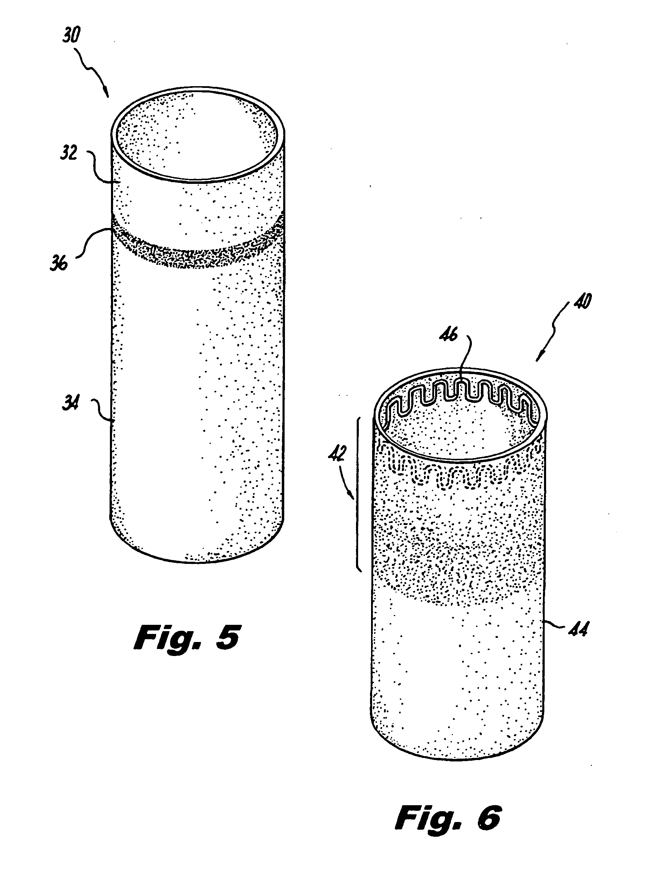

An alternate embodiment of a sheath is shown in FIG. 5. In this embodiment, a sheath 30 includes a first non-biodegradable portion 32 and a second non-biodegradable portion 34 for extending coverage of the anastomotic site. Again, non-biodegradable materials may have preferable mechanical and chemical properties. First and second non-biodegradable portions 32, 34 may be connected therebetween by a biodegradable portion 36. That is, sheath 30, which is similar to sheath 20, but sheath 30 includes the second non-biodegradable portion 34 attached to a distal end of the biodegradable portion 36. Once the anastomosis has healed, the biodegradable portion 36 will have at least partially degraded and the second non-biodegradable portion 34 can be passed naturally through the body (along with any remaining biodegradable portion 36, if any). Similarly to sheath 20, the first non-biodegradable portion 32 may later be removed through surgical means or it may detach from the body lumen wall and naturally pass through the body.

In yet another embodiment, the majority of the total length of the sheath is non-biodegradable. For example, the two non-biodegradable portions 32, 34 mentioned above with respect to sheath 30 may combine to comprise a majority of the total length of the sheath 30. In other embodiments, the majority of the length of the sheath may be comprise biodegradable portions.

Stents, by way of example, are shown as a possible attaching method in the embodiment of a sheath 40 shown in FIG.6, it being understood that other attachment methods may be employed. The proximal end of a non-biodegradable portion 42 of the sheath 40 may utilize a biodegradable stent 46, which expands and mechanically couples the sheath 40 to a body lumen wall. The stent 46 may be located interior to the non-biodegradable portion 42 or molded within a laminate structure of the non-biodegradable portion 42. In certain embodiments, the stent 46 may be self-expanding. The stent 46 may also be used in combination with a stent deployment system. When the stent 46 expands, it exerts radial force against the body lumen wall, mounting the sheath 40 in place. In alternate embodiments, a non-biodegradable stent may be employed. Once the biodegradable stent 46 loses strength or mass, the non-biodegradable portion 42 may then be released from the lumen wall and expelled through the body. It should be understood that although the above described embodiment is described to mount a non-biodegradable portion 42 of the sheath 40 to a body lumen wall, at a position spaced apart from an anastomotic site, the stent 46 can be used to couple a biodegradable portion 44.

In certain embodiments, for example, when the sheath is used in the intestinal tract, it is desirable to have the sheath flexible enough so as to allow the peristaltic motions of the intestines to effect movement of food through the composite sheath. However, there should be enough friction between the sheath and gastrointestinal tract so that peristalsis will act to straighten the sheath and apply a small amount of tension to keep the sheath in place.

Preferably, the sheath has a proper balance of mechanical properties such that the sheath maintains coverage over the anastomosis while extending distally into the body lumen. The sheath preferably maintains a certain amount of rigidity such that the sheath does not climb proximally or fold on itself, exposing the anastomosis site. In alternate embodiments, the surface of the sheath may be configured with small bumps or other surface features which will enhance the friction between the sheath and the body lumen.
It should be noted that the present disclosure is not limited to use with colonic and intestinal anastomoses and contemplates use at other anastomotic sites such as vascular anastomoses. Additionally, the above description contains many specifics; these specifics should not be construed as limitations on the scope of the disclosure herein but merely as exemplifications of particularly useful embodiments thereof. Those skilled in the art will envision many other possibilities within the scope of the disclosure as defined by the claims appended hereto.

## Claims

1. A sheath (20) for extension through an anastomotic site comprising a sleeve defining a passage, the sleeve including at least one biodegradable portion and at least one non-biodegradable portion (22), the at least one non-biodegradable portion being capable of being affixed to a tissue body proximal to an anastomotic site, and the at least one biodegradable portion (24) extending distally from the non-biodegradable portion, wherein the sheat further comprises means for attaching the at least one non biodegradable portion to a body lumen such that the sheath is capable of removal from the body lumen.

2. The sheath of claim 1, wherein the sheath further comprises a second non-biodegradable portion (34), which attaches to a distal end of the biodegradable portion.

3. The sheath of claim 2, wherein the second non-biodegradable portion extends distally from the anastomotic site.

4. The sheath of claim 3, wherein the majority of the total length of the sheath comprises the at least one non-biodegradable portion.

5. The sheath of any of claims 1 to 4, wherein said means for attaching the at least one non biodegradable portion to a body lumen are biodegradable means.

## Patentansprüche

1. Eine Scheide (20) zur Erstreckung durch eine anastomotische Stelle, umfassend eine Hülse, welche einen Durchgang definiert, wobei die Hülse wenigstens einen biologisch abbaubaren Abschnitt und wenigstens einen nicht biologisch abbaubaren Abschnitt (22) umfasst, wobei der wenigstens eine nicht biologisch abbaubare Abschnitt, mit einem Gewebekörper proximal zu einer anastomotischen Stelle verbunden werden kann, und wobei sich der wenigstens eine nicht biologisch abbaubare Abschnitt (24) distal von dem nicht biologisch abbaubaren Abschnitt erstreckt, wobei die Scheide ferner Mittel zum Anbringen des wenigstens einen nicht biologisch abbaubaren Abschnittes an einem Körperlumen umfasst, derart, dass die Scheide von dem Körperlumen entfernbar ist.

2. Scheide nach Anspruch 1, wobei die Scheide ferner einen zweiten nicht biologisch abbaubaren Abschnitt (34) umfasst, der an einem distalen Ende des biologisch abbaubaren Abschnittes befestigt ist.

3. Scheide nach Anspruch 2, wobei sich der zweite nicht biologisch abbaubare Abschnitt distal von der anastomotischen Stelle erstreckt.

4. Scheide nach Anspruch 3, wobei die Mehrheit der Gesamtlänge der Scheide den wenigstens einen nicht biologisch abbaubaren Abschnitt umfasst.

5. Scheide nach einem der vorangehenden Ansprüche 1 bis 4, wobei die Mittel zum Befestigen des wenigstens einen nicht biologisch abbaubaren Abschnittes an einem Körperlumen biologisch abbaubare Mittel sind.

## Revendications

1. Gaine (20) pour extension à travers un site d'anastomose, comprenant un manchon définissant un passage, le manchon comprenant au moins une portion biodégradable et au moins une portion non biodégradable (22), la au moins une portion non biodégradable étant à même d'être fixée à un corps de tissu proximal d'un site d' anastomose et la au moins une portion biodégradable (24) s'étendant de manière distale de la portion non biodégradable, dans laquelle la gaine comprend en outre des moyens pour fixer la au moins une portion non biodégradable à une lumière corporelle de sorte que la gaine puisse être retirée de la lumière corporelle.

2. Gaine selon la revendication 1, dans laquelle la gaine comprend en outre une seconde portion non biodégradable (34) qui se fixe à une extrémité distale de la portion biodégradable.

3. Gaine selon la revendication 2, dans laquelle la seconde portion non biodégradable s'étend de manière distale du site d'anastomose.

4. Gaine selon la revendication 3, dans laquelle la majeure partie de la longueur totale de la gaine comprend la au moins une portion non biodégradable.

5. Gaine selon l'une quelconque des revendications 1 à 4, dans laquelle lesdits moyens permettant de fixer la au moins une portion non biodégradable à une lumière corporelle sont des moyens biodégradables.
